# EUROPEAN PATENT APPLICATION

(11) **EP 4 059 596 A1**
(43) Date of publication of application: **21.09.2022**
(21) Application number: 21162811.0
(22) Date of filing: 16.03.2021
(51) Int. Cl.: B01J 8/24, B01D 17/02, C07C 29/151, C07C 31/04

(54) **PROCESS FOR METHANOL PRODUCTION FROM CO2 WITH WATER REMOVAL**

(71) Applicant: Paul Scherrer Institut, 5232 Villigen PSI (CH)
(72) Inventor: SCHILDHAUER, Tilman J., 5200 Brugg (CH); MOIOLI, Emanuele, 5200 Brugg (CH)
(74) Representative: Fischer, Michael

(57) **Abstract**

This objective is achieved according to the present invention by a process for the catalytic production of methanol from a gas mixture of at least H₂ and CO and/or CO₂ in at least one reactor, comprising the steps of:
i) bringing the gas mixture at suitable reaction temperature, pressure and gas flow conditions within the at least one reactor into contact with a catalyst and/or a solid sorbent particles for the generation of at least water and methanol and absorbing water and/or methanol and/or derivate molecules on the solid sorbent particles in order to achieve in-situ or intermediate ex-situ at least partial removal of water and/or methanol and/or derivate molecules from the flow of the gas mixture,
ii) separating at least partially at least one of the reaction products in the reactor or between a number of subsequent reactors at a temperature close to the reaction temperature by extracting the particles of the solid sorbent from the gas flow; and
iii) operating at least one of the reactors as fluidized bed reactor.

Thus, this process achieves a simple, economically more feasible process for the generation of methanol at small scale thanks to i) an efficient removal of the heat of the reaction and to control the temperature profile; and ii) overcoming the equilibrium limitation by different means than high pressure or frequent (in series or due to recycles) intermittent steps of cooling, condensation (or scrubbing) and re-heating. The extraction of the loaded solid sorbent particle and the possibility to recycle the solid sorbent particles therefore opens all possibilities to control the heat removal from the reactor(s), the condensation of water and/or methanol and/or derivate molecules on the solid sorbent particles and the re-heating of the reactor by the recycling of freshly desorbed solid sorbent particles into the reactor(s).

## Description

The present invention relates to a process for methanol production from CO₂ with water removal.

Methanol production from CO₂ is a promising route for the conversion of CO₂ into valuable products. To obtain renewable methanol, the hydrogen needed for this synthesis has to be generated renewably, e.g. obtained by converting, by electrolysis, electricity from wind or PV power plants, in particular excess electricity from those sources. Differently from the state of the art methanol production, which usually relies on natural gas supply, the capacity of production plants for renewable methanol will be limited by the local supply of either H₂ or CO₂. This is, for example, the case of biogas plants, where a limited amount of biogenic CO₂ is available for the reaction.

Renewable methanol can be used both as a CO₂-neutral fuel in engines or as an additive for gasoline. Furthermore, it is an important feedstock for organic synthesis and can be used as storage molecule for hydrogen. The reaction to form methanol from CO₂ is exothermic and limited by thermodynamics and thus requires an appropriate reactor design to achieve high yield. It is generally conducted on a Cu/ZnO-based catalyst, at pressure between 50-100 bar. The product is liquid at room temperature and pressure and can be easily separated from the unreacted gases. Being stored in liquid form, methanol is simply transported from the production site to the final consumers.

The main challenge in the methanol production from CO₂ is the strict thermodynamic limit, which requires to avoid hotspots and to keep the temperature at the reactor end low and/or even constant over the complete reactor. In order to do so, the heat transfer must be kept as high as possible in the entire reactor.

A suitable reactor concept for this scope is the fluidized bed reactor, due to the continuous back mixing caused by the catalyst in continuous movement in the reactor.

The state of the art methanol synthesis is achieved in fixed bed catalytic reactors composed of several pipes filled with a Cu/ZnO/Al₂O₃ catalyst. The heat production of the reaction is controlled by adaptation of the heat transfer on the cooling side or by use of intermediate quenching and split feed. Further, often high pressures and recirculation of unreacted compounds is applied. Due to economic reasons, these solutions cannot, however, easily be implemented at low plant capacities, that could be requested by distributed methanol production, made possible by the local availability of renewable electrical energy and by the local CO₂ sources, for example from biogas, sewage sludge and gasification.

Several processes or concepts that allow methanol production from CO₂ or CO have been published, including one or more reactors in series. Some report the use of cascade fluidized reactors to produce methanol from syngas. The process concerns the production of methanol by reaction of a gaseous mixture composed of hydrogen and carbon monoxide in the presence of a catalyst composition in a fluidized bed at 50 to 350 bar. This reactor is externally cooled and the process is characterized by the presence of a plurality of fluidized catalyst bed reactors in series with interstage removal of methanol from the reaction mixture by condensation or adsorption/absorption with a solvent. Further, the use of a fast fluidized bed reactor is reported to produce process methanol from a synthesis gas (syngas) feed. The reactor is operated at substantially plug flow type behavior. The catalyst is circulated so that the heat is captured by the catalyst and the reaction can be initiated with little to no preheating of feed. In addition, little reactor cooling is needed. In the later the process refers to the production of the methanol product from synthesis gas (syngas) using a fluid bed reactor. Back mixing of feed and catalyst is used to balance the internal reactor heat transfer. The back mixing is controlled with a series of control points, among them superficial gas velocity, catalyst density in the reactor, reactor height to diameter ratio (especially in the dense catalyst bed region) and catalyst particle size.

Others design a process in which methanol is produced via exothermic equilibrium reactions and methanol, water and heat are partially removed from the methanol synthesis reactor or at an intermediate point between two or more methanol synthesis reactors by means of a solvent. This solvent can absorb methanol, water and heat. The preferred reactor system comprises a first boiling water reactor and a successive reactor for the synthesis of methanol. With this method, it is possible to obtain a sufficient temperature control across the catalytic bed. Solvent can be an alcohol, such as propylene glycol or glycerol.

Several patents/patent applications concern the production of methanol with continuous product removal by means of a liquid absorbent. They report a process composed of one or more fixed-bed reactors in which a liquid absorbent removes the methanol formed. The liquid absorbent is then regenerated in a separated unit. Some design systems for the absorption-enhanced synthesis in equilibrium-limited reactions. These systems include an appropriate reactor design to guarantee the circulation of the liquid absorbent in the reactor and the removal of the liquid product.

Others disclose a system composed of a plurality of fluidized bed reactors in series for the synthesis of methanol. The reactants are co-fed with an inert solvent that absorbs the reaction product. The solvent can be water, a higher alcohol, a higher ether or a higher ester. The liquid mixture containing the product is separated after the reactor and the gaseous reactants are fed to the next reaction stage.

There is also a system for the continuous production of methanol by use of simulated moving-bed reactive chromatography. With this system, the methanol produced is adsorbed on a proper material, which is progressively moved through the reactor through a simulated moving bed. Additional disclosure provides for a pressure swing adsorption (PSA) process for the performance of reversible reactors. In this case, methanol is produced in the PSA unit, in the presence of a uniformly distributed adsorbent. The product is adsorbed at the reaction temperature and pressure. The sorbent is then regenerated with various strategies to obtain the final product.

Others design a slurry bed reactor for increasing the yield of equilibrium-limited reactions by means of co-feed of an absorbing agent. The absorbing agent reacts with one or more products and it is transmitted to a recycle or a regeneration unit. The absorbing agent can also be replaced by fresh material in a replacing unit. In the prior art, one also finds a system for carrying out equilibrium reactions in a two-stage process. In the first step, the process parameters are adjusted to have both reactants and products in gas phase. In the second step, the reaction is performed in the presence of an adsorbent, which captures one of the products. The adsorbed methanol is then separated in a desorption unit. Adsorbing agent and unreacted components are recycled back to the first reactor.

Further, a system for the production of ammonia at high conversion rates in a fluidized bed reactor is known. Sorbent particles being at least ten times smaller than the catalyst particle are co-fed in the reactor. The sorbent particle selectively remove ammonia from the reactor and are regenerated in a special unit. A reactor system including a fluidized bed reactor including sorbent particles that are entrained in the product stream is known, so that they can be removed at the reactor outlet and regenerated. The catalytic particles are sized to stay gravimetrically in the fluidized zone.

It is therefore the objective of the present invention to provide technical solutions to make the small-scale methanol production from CO₂ possible, achieving high conversion in a simple system. Especially the development of high pressures is limited as the options for internal energy recovery are significantly smaller than in state-of-art systems where the off-heat from the natural gas reformer is used to raise steam which then is fed to a turbine which drives a gas compressor on the same axis.

This objective is achieved according to the present invention by a process for the catalytic production of methanol from a gas mixture of at least H₂ and CO and/or CO₂ in at least one reactor, comprising the steps of:
i) bringing the gas mixture at suitable reaction temperature, pressure and gas flow conditions within the at least one reactor into contact with a catalyst and/or a solid sorbent particles for the generation of at least water and methanol and absorbing water and/or methanol and/or derivate molecules on the solid sorbent particles in order to achieve in-situ or intermediate ex-situ at least partial removal of water and/or methanol and/or derivate molecules from the flow of the gas mixture,
ii) separating at least partially at least one of the reaction products in the reactor or between a number of subsequent reactors at a temperature close to the reaction temperature by extracting the particles of the solid sorbent from the gas flow; and
iii) operating at least one of the reactors as fluidized bed reactor.

Thus, this process achieves a simple, economically more feasible process for the generation of methanol at small scale thanks to i) an efficient removal of the heat of the reaction and to control the temperature profile; and ii) overcoming the equilibrium limitation by different means than high pressure or frequent (in series or due to recycles) intermittent steps of cooling, condensation (or scrubbing) and re-heating. The extraction of the loaded solid sorbent particle and the possibility to recycle the solid sorbent particles therefore opens all possibilities to control the heat removal from the reactor(s), the condensation of water and/or methanol and/or derivate molecules on the solid sorbent particles and the re-heating of the reactor by the recycling of freshly desorbed solid sorbent particles into the reactor(s).

Advantageous features which can be used in suitable combinations are listed below:
i) a regeneration of the solid sorbent is conducted within at least one of the reactor(s) by a temperature increase or an addition of dry gases or a combination thereof;
ii) the regeneration of the solid sorbent is conducted outside the reactor(s) by a temperature increase and/or a pressure decrease and/or addition of dry gases;
iii) at least one further line for the flow of the gas mixture is available to allow switchable operation for uninterrupted generation of methanol and an alternating regeneration of the solid sorbent;
iv) the heat of the methanol reaction in one production line is at least partially used for the regeneration of the solid sorbent in the other line(s);
v) a combination of separation methods, such as the size and specific weight of solid sorbents, fluids with low vapor pressure, with different properties is applied to at least partly enrich the materials used in this separation method during ad-/absorption with the reaction products;
vi) at least one of the reactors are operated with catalysts based on metals like Cu, In, Ir, Ag, Pd, Pt, Zn, Ga or their oxides supported on Al2O3, SiO2, TiO2, CeO2, ZrO2, carbides, nitrides, phosphides or a mixture thereof or a perovskite type material containing one of the mentioned elements;
vii) water and/or methanol or other compounds are at least partially removed in-situ from the fluidized bed reactor by use of fine or less dense solid sorbent particles which are entrained from the reactor or floating on a reactor bed and collected downstream of the particle bed of the reactor, and wherein the solid sorbents are zeolite, alumina, silica, silicate, carbon material or mixtures thereof;
viii) water and/or methanol or other compounds are at least partially removed in-situ from the fluidized bed reactor by use of larger or more dense solid sorbent particles which are separated by gravity from the reactor and are collected at the bottom of the reactor, and wherein the solid sorbents are zeolite, alumina, silica, silicate, carbon material or mixtures thereof;
ix) water and/or methanol or other compounds are at least partially removed in-situ from the fluidized bed reactor by combined use of larger and/or more dense solid sorbent particles which are separated by gravity from the reactor and fine and/or less dense solid sorbent particles, which are entrained from the reactor or floating on the bed of the reactor;
x) water and/or methanol or other compounds are at least partially removed in-situ from the fluidized bed reactor by immersing structures containing or consisting of solid sorbent;
xi) a feed stream is composed of CO₂, biogas, gas from fermentations or sewage sludge digestion or producer gas from gasification or pyrolysis;
xii) the CO₂:H₂ ratio or CO:H₂ ratio in the feed stream is different from the stoichiometric ratio of the methanol synthesis reaction wherein an excess of reactant, not converted in the reactor(s), is at least partly recycled and mixed with the feed stream;
xiii) hydrogen is fed in stoichiometric excess;
xiv) a second catalyst or catalytic functionality is introduced into the reactor to enable follow-up reactions of methanol and other reaction products;
xv) the reactants CO, CO₂ and/or H₂ remaining after the methanol production are converted to methane and/or other hydrocarbons in an appropriate reactor, using a catalyst containing at least one of the elements Nickel, Ruthenium, Iron or Cobalt;
xvi) another gas stream is mixed with the reactants CO, CO₂ and/or H₂ remaining after the methanol production before the conversion to methane and/or other hydrocarbons in an appropriate reactor;
xvii) at least one membrane unit separates at least partly methane from the feed stream to the methanol synthesis reactor to bypass the methanol synthesis;
xviii) the CO₂ source is a biogas plant and wherein the membrane unit is used for biogas-upgrading during times when the methanol generation is not or not fully operational;
xix) the reactants CO, CO₂ and/or H₂ remaining after the methanol production are converted to electricity and heat in a suitable plant, preferably a combined heat and power plant based on an internal combustion engine or a micro gas turbine or a fuel cell; and
xx) another gas stream is mixed with the reactants CO, CO₂ and/or H₂ remaining after methanol production before conversion to electricity and heat in the suitable device.

Preferred embodiments of the present invention invention are described hereinafter in more detail with reference to the attached drawings, which depict in:
- Figure 1: schematically the process configuration for a multi reactor methanol generation process;
- Figure 2: schematically a reactor filled with a sorbent lighter than the catalyst being separated from the top of the reactor;
- Figure 3: schematically a reactor filled with a sorbent heavier than the catalyst being separated from the bottom of the reactor;
- Figure 4: a schematical representation of the process which is hereinafter explained under Example 1; and
- Figure 5: a schematical representation of the process which is hereinafter explained under Example 2.

### Concept of the invention

To achieve a simple, economically more feasible process for the generation of methanol at small scale, it is necessary to i) efficiently remove the heat of the reaction and to control the temperature profile; and ii) to overcome the equilibrium limitation by different means than high pressure or frequent (in series or due to recycles) intermittent steps of cooling, condensation (or scrubbing) and re-heating.

Clearly, catalyst active at as low temperature as possible are helpful and further adding reactants in excess and removal of reaction products are favorable.

To avoid a too high extent of the reverse water gas shift (which produces CO that in too high concentrations could deactivate the catalyst), hydrogen should be added in excess. Non-reacted hydrogen at the end of the synthesis could be separated and recycled to the methanol synthesis or can be fed to a subsequent process or reactor. In this subsequent reactor, the hydrogen and unconverted carbon oxides (CO, CO₂) could react to another useful product such as renewable methane, where it might be helpful to add another gas stream such as CO₂, biogas or producer gas from gasification, pyrolysis or similar to obtain the correct stoichiometric ratio.

Alternatively, the unreacted gas could be used thermally in a gas engine, fuel cell or gas turbine to produce electricity and heat where the heat could be used for purposes such as raising steam for high temperature electrolysis, heating of reactors or buildings, feedstock pretreatments, raising of cold by absorption refrigeration, and/or similar processes.

Removal of reaction products by condensation before the recycle is part of the state of art processes; other concepts suggested the use of solvents. In the process suggested here, the water removal should take place by solid sorbents that can take up water and/or methanol and other condensable products at temperatures close to the reaction temperature. This allows avoiding too many heat exchangers etc. needed for cooling down to the temperature level of condensation or scrubbing with solvents and subsequent re-heating.

The use of (solid) sorbents inherently means that these have to be regenerated either in-situ (by stopping the methanol synthesis and flushing with gas or by decreasing the pressure) or ex-situ by removing the sorbent from the reactor and introducing fresh or regenerated sorbent. During the in-situ regeneration, the reactant flow can be fed to a parallel second synthesis line to allow uninterrupted production. Here, the off-heat of one process line can be helpful for the regeneration the second line. For this, very close thermal contact has to be ensured, either by direct heat transfer and conduction from reactor chamber to reactor chamber or by applying suitable heat transfer media (thermo-oil loops, gas loops, heat pipes, steam cycles etc.) or by heat exchange with process streams.

Ex-situ regeneration requires removing the (nearly) spent sorbents from the process during operation, which could happen from time to time or even continuously. The moving of solid sorbents through reactors is especially facilitated in fluidized bed reactors where the catalyst and the gas together form a fluid type phase. As this reactor also comprises excellent heat transfer properties, it is especially useful for the process concept suggested here, as described in the following. In case of adding a fluid with very low vapor pressure, three phase reactors such as trickle beds (preferably in countercurrent operation) and especially bubble column could be applied as well.

### Process concept with fluidized bed reactor

CO₂ and H₂, in a ratio variable from 1:3 to 1:10, are fed into a catalytic reactor, to form at least CH₃OH and H₂O, but potentially also molecules derived from methanol such as dimethyl-ether and similar. The feed stream can also contain significant amounts of CH₄, for example because CO₂ originates from biogas. The reaction occurs on an adequate catalyst and/or catalyst combinations. In the course of the reaction, heat is produced and the cooling medium (in the immersed heat exchangers or reactor shell) removes it. The reactor could be designed as a circulating or bubbling fluidized bed. The use of this reactor type allows an efficient removal of the reaction heat.

Thanks to the large mixing, the good heat transfer is maintained independently from the gas flow rate, allowing a stable performance of the reactor. This is not possible with the standard packed bed reactors, as the heat transfer coefficient for this class of reactors is strongly dependent on the gas flow rate. The necessary long pipes of these reactors are unpractical for small scale applications. With the fluidized bed technology, a compact reactor can be designed, making this solution particularly suitable for small-scale applications.

The reactor can also be used for the production of methanol from biogas mixture. In this case, the methane present is treated as inert in the reactor and H₂ is added to the reacting mixture in an appropriate ratio to the present CO₂, varying from 1:3 to 1:10. When working with excess of hydrogen, the unreacted gases can be passed in an additional reactor, over Ni, Co or Ru-based catalyst to convert the remaining and/or additional carbon oxides (CO and CO₂) to methane.

Alternatively, unreacted gas from the methanol synthesis reactors could be converted to heat and electricity in a combined heat and power plant (CHP) such as a gas motor, (micro) gas turbine or a (high temperature) fuel cell. When using biogas as source for CO₂, it might make sense to integrate a membrane unit to separate parts of the methane before the first methanol synthesis. This lowers the dilution of the reactive gas by methane. When no renewable hydrogen is available and it makes hardly sense to operate the methanol synthesis, e.g. in winter, the membrane unit could be used for upgrading of the raw biogas to biomethane.

The classical methanol synthesis reaction requires intermediate condensation of the products in order to overcome the thermodynamic limitations. For this reason, the system may be composed of several reactors in series, with intermediate water separation steps. Water and methanol are removed in this intermediate operation by cooling and condensing the liquids or by use of an appropriate absorbing fluid or by adsorption on a specific material (solid sorbent). By choosing different methods or materials, at least partially selective removal of either water or methanol or derivate molecules such as DME could be applied to facilitate later separation of the reaction products from each other. The remaining CO, CO₂ and H₂ are fed to the subsequent reactor.

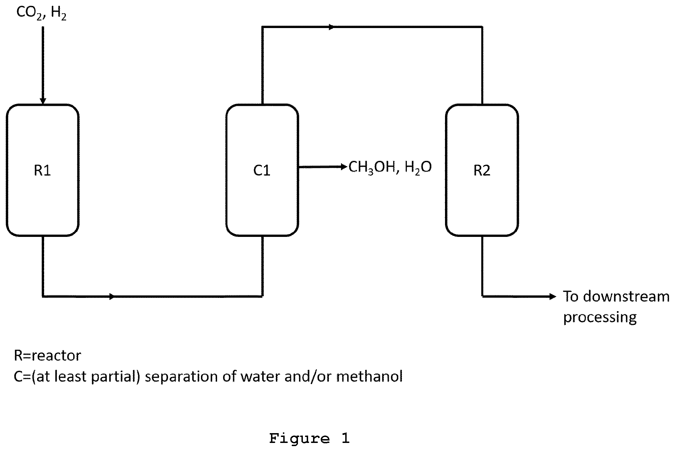

Figure 1 depicts schematically this process configuration. As the reverse water-gas-shift reaction may take place as a side reaction of the methanol synthesis, the CO₂:CO ratio is changed over the consecutive reactors, with increasing CO content in the gas stream. For this reason, the temperature of the reactor may be varied stage-by-stage, being the optimal temperature higher with a higher amount of CO present, due to the different thermodynamic equilibrium curve.

Furthermore, the fluidized bed reactor allows for a special management of the water removal. This can be obtained by a proper use of a water sorbent. The water sorbent can be composed of zeolites, alumina, silica, silicates, active carbons or other appropriate material. The sorbent can be formulated, sized or designed in a different way than the catalyst, to exploit the different fluid-dynamic properties of the materials and efficiently remove the sorbent. Two different strategies can be applied, as explained in the following.

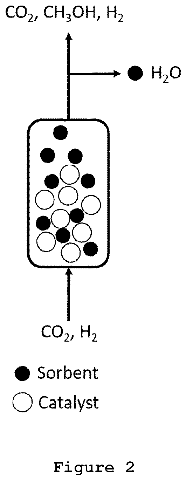

In the fluidized bed, the sorbent can be shaped in a way to have a lower fluidization region than the catalyst. In this case, two different fluid-dynamic regimes exist for the two distinguished materials in the reactor: the catalyst remains suspended in the dense phase of the fluidized bed while the sorbent results in entrained flow along the reactor. The sorbent is thus transported out of the catalytic bed by the gas flow and can be further treated at the reactor outlet. This process principles are depicted in figure 2. The sorbent can be regenerated in various ways. In one possible solution, in the upper part of the reactor an appropriate change of temperature and/or addition of dry gas can be operated to regenerate in-situ the sorbent, which is then directly recirculated to the catalytic bed. In another configuration, the sorbent is collected at the reactor top outlet and fed to a second equipment for the regeneration. This equipment is operated in a circulating bed way, either continuously or batch wise. The regeneration can proceed via treatment at a higher temperature, lower pressure, via drying in a suitable process stream or a combination thereof.

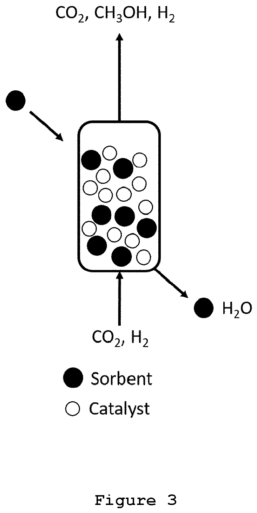

Another sorbent configuration allows a different separation from the catalyst. If the sorbent shows a fluidization region at significantly higher flow rates than the catalyst (e.g. because the sorbent particles are bigger than the catalyst particles or of sufficiently higher density), this separates the sorbend particles from the catalyst particles in the dense phase precipitating on the bottom of the reactor. This phenomenon can be used to design an in-situ liquid removal strategy as explained in figure 3. The sorbent is fed from the top of the reactor and progressively descends the reactor towards the bottom, while collecting liquids. The spent sorbent is then collected, regenerated and fed again on the top of the reactor. In a third variation, structures containing or consisting of solid sorbent (e.g. rods or particles in permeable bags or nets) are immersed into the fluidized bed and removed for the regeneration, either in situ (above the bed with raised temperature and/or dry gas flow) or ex-situ.

### Example 1

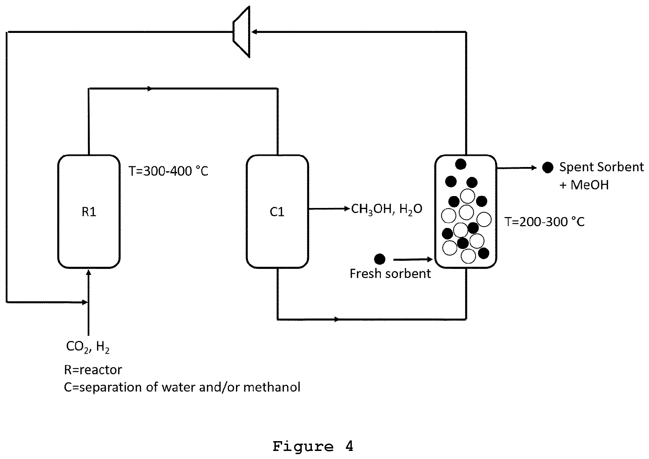

A mixture of CO₂ and H₂ in ratio 3:1 is fed to a first fluidized bed reactor R1. This reactor is operated between 200-300 °C and between 20-50 bar. The catalyst used is Cu/ZnO/Al₂O₃. The product gases from this reactor are passed through a condenser C1, where the temperature is reduced to 150 °C and the liquid products are separated. The remaining gas mixture, composed of CO, CO₂ and H₂ is fed to the second fluidized bed reactor. This second reactor is operated between 200-300 °C and in the presence of a zeolite-based sorbent. The sorbent is continuously fed with the reacting gases from the bottom of the second reactor and entrained by the gas flow. The spent sorbent is separated from the product gases at the top of the reactor by a cyclone, sent to an appropriate regeneration unit and recycled to the reactor. The unreacted gases are recycled to the first reactor after recompression. The process principles of Example 1 are schematically summarized in figure 4.

### Example 2

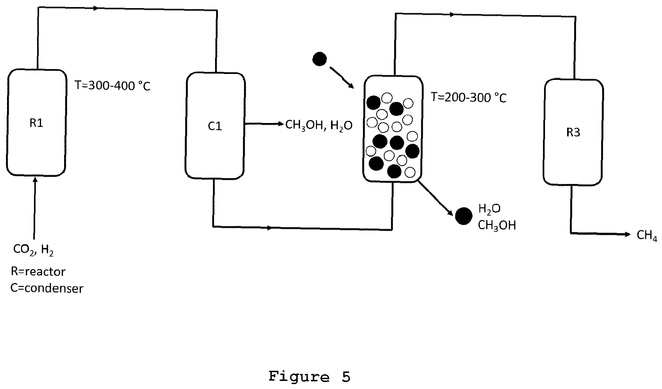

A biogas mixture, composed of CO₂ and CH₄, is enriched in H₂, to a stoichiometric ratio CO₂:H₂ higher than 3:1 is fed to a first fluidized bed reactor R1 operating at 200-400 °C over a Cu/ZnO/Al₂O₃ catalyst. The product liquids of this first reactor are eliminated in an appropriate unit by condensation C1. The remaining gas, composed of CO, CO₂ CH₄ and H₂, is fed to the second methanol reactor, where it is also in contact, in countercurrent, with a zeolite-based sorbent. The sorbent extracts in-situ the product water and methanol and it is collected from the bottom of the second reactor, regenerated and fed back to the second reactor. The remaining unreacted gases are fed to a methanation reactor R3, where the remaining CO and CO₂ is converted to methane. A schematic representation of this process is depicted in figure 5.

### Reviewed prior art

US 5,216,034; US 2007/0299146; US 7,579,383 B2; WO 2016/096425 A1; EP 0 326 718 A1; WO 2016/128187 A1; EP 3 492 165 A1; GB2255516A; US005523326A; US 20120232174 A1; GB2293334A; WO 2018/136849 A1 and US 20100092381A1..

## Claims

1. A process for the catalytic production of methanol from a gas mixture of at least H₂ and CO and/or CO₂ in at least one reactor, comprising the steps of:
i) bringing the gas mixture at suitable reaction temperature, pressure and gas flow conditions within the at least one reactor into contact with a catalyst and/or a solid sorbent particles for the generation of at least water and methanol and absorbing water and/or methanol and/or derivate molecules on the solid sorbent particles in order to achieve in-situ or intermediate ex-situ at least partial removal of water and/or methanol and/or derivate molecules from the flow of the gas mixture,
ii) separating at least partially at least one of the reaction products in the reactor or between a number of subsequent reactors at a temperature close to the reaction temperature by extracting the particles of the solid sorbent from the gas flow; and
iii) operating at least one of the reactors as fluidized bed reactor.

2. The process according to claim 1, wherein a regeneration of the solid sorbent is conducted within at least one of the reactor(s) by a temperature increase or an addition of dry gases or a combination thereof.

3. The process according to claim 1 or 2, wherein the regeneration of the solid sorbent is conducted outside the reactor(s) by a temperature increase and/or a pressure decrease and/or addition of dry gases.

4. The process according to any of the preceding claims wherein at least one further line for the flow of the gas mixture is available to allow switchable operation for uninterrupted generation of methanol and an alternating regeneration of the solid sorbent.

5. The process according to claim 4, wherein the heat of the methanol reaction in one production line is at least partially used for the regeneration of the solid sorbent in the other line(s).

6. The process according to any of the preceding claims, wherein a combination of separation methods, such as the size and specific weight of solid sorbents, fluids with low vapor pressure, with different properties is applied to at least partly enrich the materials used in this separation method during ad-/absorption with the reaction products.

7. The process according to any of the preceding claims, wherein at least one of the reactors are operated with catalysts based on metals like Cu, In, Ir, Ag, Pd, Pt, Zn, Ga or their oxides supported on Al2O3, SiO2, TiO2, CeO2, ZrO2, carbides, nitrides, phosphides or a mixture thereof or a perovskite type material containing one of the mentioned elements.

8. The process according to any of the preceding claims, wherein water and/or methanol or other compounds are at least partially removed in-situ from the fluidized bed reactor by use of fine or less dense solid sorbent particles which are entrained from the reactor or floating on a reactor bed and collected downstream of the particle bed of the reactor, and wherein the solid sorbents are zeolite, alumina, silica, silicate, carbon material or mixtures thereof.

9. The process according to any of the preceding claims 1 to 7, wherein water and/or methanol or other compounds are at least partially removed in-situ from the fluidized bed reactor by use of larger or more dense solid sorbent particles which are separated by gravity from the reactor and are collected at the bottom of the reactor, and wherein the solid sorbents are zeolite, alumina, silica, silicate, carbon material or mixtures thereof.

10. The process according to any of the preceding claims 1 to 7, wherein water and/or methanol or other compounds are at least partially removed in-situ from the fluidized bed reactor by combined use of larger and/or more dense solid sorbent particles which are separated by gravity from the reactor and fine and/or less dense solid sorbent particles, which are entrained from the reactor or floating on the bed of the reactor.

11. The process according to any of the preceding claims 1 to 7, wherein water and/or methanol or other compounds are at least partially removed in-situ from the fluidized bed reactor by immersing structures containing or consisting of solid sorbent.

12. The process according to any of the preceding claims wherein a feed stream is composed of CO₂, biogas, gas from fermentations or sewage sludge digestion or producer gas from gasification or pyrolysis.

13. The process according to any of the preceding claims, wherein the CO₂:H₂ ratio or CO:H₂ ratio in the feed stream is different from the stoichiometric ratio of the methanol synthesis reaction wherein an excess of reactant, not converted in the reactor(s), is at least partly recycled and mixed with the feed stream.

14. The process according to claim 13 wherein hydrogen is fed in stoichiometric excess.

15. The process according to any of the preceding claims, wherein a second catalyst or catalytic functionality is introduced into the reactor to enable follow-up reactions of methanol and other reaction products.

16. The process according to any of the preceding claims, wherein the remaining reactants CO, CO₂ and/or H₂ after the methanol production are converted to methane and/or other hydrocarbons in an appropriate reactor, using a catalyst containing at least one of the elements Nickel, Ruthenium, Iron or Cobalt.

17. The process according to claim 16, wherein another gas stream is mixed with the reactants CO, CO₂ and/or H₂ remaining after the methanol production before the conversion to methane and/or other hydrocarbons in an appropriate reactor.

18. The process according to claim 16 or 17, wherein at least one membrane unit separates at least partly methane from the feed stream to the methanol synthesis reactor to bypass the methanol synthesis.

19. The process according to claim 18, wherein the CO₂ source is a biogas plant and wherein the membrane unit is used for biogas-upgrading during times when the methanol generation is not or not fully operational.

20. The process according to any of the preceding claims 1 to 15, wherein the reactants CO, CO₂ and/or H₂ remaining after the methanol production are converted to electricity and heat in a suitable plant, preferably a combined heat and power plant based on an internal combustion engine or a micro gas turbine or a fuel cell.

21. The process according to claim 20, wherein another gas stream is mixed with the reactants CO, CO₂ and/or H₂ remaining after methanol production before conversion to electricity and heat in the suitable device.
